(19) Europäisches Patentamt / European Patent Office / Office européen des brevets

(11) EP 1 897 564 A1

(12) **DEMANDE DE BREVET EUROPEEN**

(43) Date de publication:
**12.03.2008 Bulletin 2008/11**

(51) Int Cl.:
***A61L 2/20*** *(2006.01)* ***A61L 9/04*** *(2006.01)*
***C07C 39/28*** *(2006.01)*

(21) Numéro de dépôt: **07356117.7**

(22) Date de dépôt: **03.09.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **04.09.2006 FR 0607736**

(71) Demandeur: **Vasseur, Pierre Roger**
**78670 Villennes sur Seine (FR)**

(72) Inventeur: **Vasseur, Pierre Roger**
**78670 Villennes sur Seine (FR)**

(74) Mandataire: **Martin, Didier Roland Valéry**
**Cabinet Didier Martin**
**50, chemin des Verrières**
**69260 Charbonnières-les Bains (FR)**

(54) **Dispositif de désinfection par voie aérienne, substance et procédés correspondants**

(57) L'invention concerne un dispositif de désinfection par voie aérienne (1) comprenant d'une part une substance désinfectante (2) et, d'autre part, un moyen de diffusion (3) conçu pour diffuser par évaporation naturelle ladite substance désinfectante (2) dans l'atmosphère qui environne ledit dispositif (1), caractérisé en ce que ladite substance désinfectante (2) comprend un principe actif phénolique.

Dispositifs de désinfection.

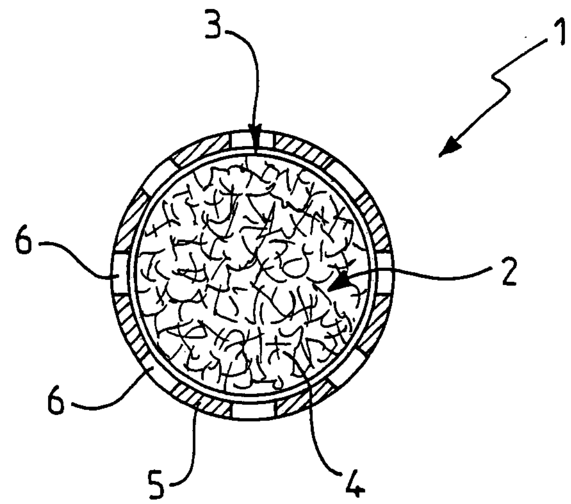


FIG.2

EP 1 897 564 A1

## Description

La présente invention se rapporte aux dispositifs de désinfection par voie aérienne capables d'agir à distance sans nécessiter de contact direct avec les surfaces à traiter.

Plus particulièrement, la présente invention concerne un dispositif de désinfection par voie aérienne comprenant d'une part une substance désinfectante et, d'autre part, un moyen de diffusion conçu pour diffuser par évaporation naturelle ladite substance désinfectante dans l'atmosphère qui environne ledit dispositif.

La présente invention concerne également une substance désinfectante conçue pour être diffusée dans l'atmosphère par évaporation naturelle.

La présente invention concerne également un procédé de fabrication d'un dispositif de désinfection par voie aérienne dans lequel on associe une substance désinfectante à un moyen de diffusion conçu pour diffuser par évaporation naturelle ladite substance désinfectante dans l'atmosphère qui environne ledit dispositif.

La présente invention concerne enfin un procédé d'élaboration d'une substance désinfectante conçue pour être diffusée dans l'atmosphère par évaporation naturelle.

Il est connu d'employer des dispositifs de désinfection par voie aérienne qui agissent en diffusant dans l'atmosphère un principe actif en phase gazeuse ou *« phase vapeur ».* De tels dispositifs de l'art antérieur exercent avantageusement une action fongicide et/ou bactéricide sur les surfaces d'objets à traiter qui sont placés dans leur environnement.

Plus particulièrement, il est connu d'employer de tels dispositifs sous la forme de sphères de diffusion destinées à désinfecter des articles vestimentaires tels que des chaussures. L'objectif d'une telle désinfection est double, puisque la suppression des bactéries et champignons constitue à la fois une mesure d'hygiène et un moyen de prévenir l'apparition de mauvaises odeurs en éliminant les micro-organismes qui en sont à l'origine.

Bien qu'ils présentent des résultats satisfaisants en matière de lutte contre les micro-organismes indésirables, les dispositifs de l'art antérieur souffrent cependant d'inconvénients non négligeables.

En effet, les principes actifs employés dans les dispositifs de l'art antérieur sont généralement des composés qui présentent une ou plusieurs fonctions ammonium quaternaire, tels que le benzylcoco alkyldiméthyl ammonium chlorure, mieux connu sous l'appellation commerciale *« Rhodoquat RP 80 ®».*

Or, de tels principes actifs, s'ils donnent par ailleurs toute satisfaction, sont susceptibles, dans certaines conditions, de présenter une toxicité vis-à-vis de l'utilisateur et de l'environnement, ce qui conduit naturellement à en restreindre l'utilisation, voire à en interdire la commercialisation par voie réglementaire (cf. directives européennes 98/8/CE sur les produits biocides).

Les objets assignés à l'invention visent par conséquent à porter remède aux inconvénients mentionnés précédemment et à proposer un nouveau dispositif de désinfection par voie aérienne qui permette une désinfection efficace, tout en étant respectueux de l'environnement et de la santé de l'utilisateur.

Un autre objet de l'invention vise à proposer un nouveau dispositif de désinfection qui soit particulièrement simple à fabriquer et qui présente un prix de revient compétitif.

Un autre objet de l'invention vise à proposer un nouveau dispositif de désinfection qui soit de conception simple et compacte.

Un autre objet de l'invention vise à proposer un nouveau dispositif de désinfection dont la longévité soit optimisée.

Un autre objet de l'invention vise à proposer une nouvelle substance désinfectante qui présente une action efficace par voie aérienne, tout en étant respectueuse de l'environnement et de la santé de l'utilisateur.

Un autre objet de l'invention vise à proposer une nouvelle substance désinfectante dont l'élaboration soit particulièrement simple et peu coûteuse, et dont la mise en oeuvre soit particulièrement aisée.

Un autre objet de la présente invention vise à proposer un nouveau procédé de fabrication d'un dispositif de désinfection par voie aérienne qui permette d'obtenir un dispositif de désinfection efficace et compatible avec les normes environnementales et sanitaires en vigueur.

Un autre objet de la présente invention vise à proposer un procédé de fabrication d'un dispositif de désinfection dont la mise en oeuvre soit simple et économiquement compétitive.

Enfin, un autre objet de la présente invention vise à proposer un nouveau procédé d'élaboration d'une substance désinfectante qui présente une action efficace par voie aérienne, tout en étant respectueuse de l'environnement et de la santé de l'utilisateur.

Les objets assignés à l'invention sont atteints à l'aide d'un dispositif de désinfection par voie aérienne comprenant d'une part une substance désinfectante et, d'autre part, un moyen de diffusion conçu pour diffuser par évaporation naturelle ladite substance désinfectante dans l'atmosphère qui environne ledit dispositif, caractérisé en ce que ladite substance désinfectante comprend un principe actif phénolique.

Les objets assignés à l'invention sont également atteints à l'aide d'une substance désinfectante conçue pour être diffusée dans l'atmosphère par évaporation naturelle, caractérisée en ce qu'elle comprend un principe actif phénolique.

Les objets assignés à l'invention sont également atteints à l'aide d'un procédé de fabrication d'un dispositif de désinfection par voie aérienne dans lequel on associe une substance désinfectante à un moyen de diffusion conçu pour diffuser par évaporation naturelle ladite substance désinfectante dans l'atmosphère qui environne ledit dispositif, caractérisé en ce que la substance désinfectante employée comprend un principe actif phénolique.

Les objets assignés à l'invention sont enfin atteints à l'aide d'un procédé d'élaboration d'une substance désinfectante conçue pour être diffusée dans l'atmosphère par évaporation naturelle, caractérisé en ce qu'il comporte une étape au cours de laquelle on réalise une préparation comprenant un principe actif phénolique à partir de laquelle on fabrique ladite substance désinfectante.

D'autres caractéristiques et avantages de l'invention apparaîtront plus en détails à la lumière de la description qui suit, ainsi qu'à l'aide des dessins annexés fournis à titre illustratif et non limitatif, parmi lesquels :

- La figure 1 représente, selon une vue en perspective, une variante de réalisation d'un dispositif de désinfection conforme à l'invention.

- La figure 2 illustre, selon une vue en coupe sagittale selon le plan P1, le dispositif de la figure 1.

Le dispositif de désinfection par voie aérienne conforme à l'invention est destiné à exercer une action désinfectante, et plus particulièrement bactéricide et/ou fongicide, sur divers objets sans entrer directement en contact avec les surfaces à traiter.

Le dispositif de désinfection 1 conforme à l'invention se distingue donc des dispositifs de désinfection au contact tels que les systèmes d'aspersion ou d'immersion.

A cet effet, le dispositif de désinfection 1 conforme à l'invention comprend d'une part une substance désinfectante 2 et, d'autre part, un moyen de diffusion 3 conçu pour diffuser par évaporation naturelle ladite substance désinfectante 2 dans l'atmosphère qui environne ledit dispositif 1.

Ainsi, la substance désinfectante 2 est apte à exercer son action bactéricide et/ou fongicide alors qu'elle se trouve à l'état gazeux, c'est à dire en phase vapeur dans l'atmosphère dans laquelle baigne l'objet à traiter.

Par *« évaporation naturelle »*, on désigne le phénomène de libération ou de passage en phase vapeur de la substance désinfectante 2 selon un phénomène naturel de diffusion. Au sens de l'invention, l'évaporation naturelle s'oppose donc à une évaporation « *forcée* », qui impliquerait la mise en oeuvre d'un mécanisme thermique tel que le chauffage et/ou chimique par la création d'un produit de réaction gazeux (notamment par combustion).

Ainsi, la substance désinfectante 2 conforme à l'invention est avantageusement apte à se vaporiser spontanément et continûment à la température normale de mise en oeuvre du dispositif, c'est à dire de préférence à température ambiante.

Selon une caractéristique importante de l'invention, la substance désinfectante 2 comprend un principe actif phénolique, c'est à dire une molécule qui porte au moins un groupement phénol.

De façon préférentielle, le principe actif phénolique est à base de 3-méthyl-4-chlorophénolate de sodium, dont la formule est donnée ci-dessous :

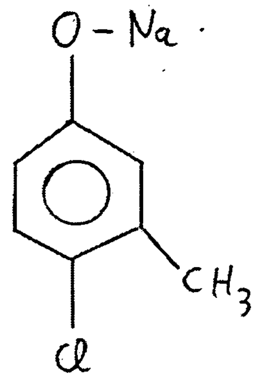

*Formule 1 : 3-méthyl-4-chlorophénolate de sodium*

Avantageusement, le principe actif phénolique confère à la substance désinfectante 2 une grande efficacité

d'action à la fois bactéricide et fongicide, tout en étant plus respectueux de l'environnement et de la santé des utilisateurs que les composés d'ammonium quaternaire de l'art antérieur.

En particulier, le 3-méthyl-4-chlorophénolate de sodium présente une quasi innocuité qui le rend particulièrement adapté à une utilisation grand public.

De préférence, le principe actif phénolique conforme à l'invention est formé majoritairement et, de façon encore plus préférentielle, quasi exclusivement de 3-méthyl-4-chlorophénolate de sodium.

Plus particulièrement, ce principe actif phénolique pourra être approvisionné sous la forme d'une préparation disponible dans le commerce connue sous le nom commercial de *« PREVENTOL CMK-NA®* », ci-après « *Préventol®* », qui contient usuellement 71 % à 73% en poids de 3-méthyl-4-chlorophénolate de sodium.

Dans ce qui suit, on considérera par commodité de description que le principe actif phénolique est formé uniquement par du Préventol®, sans que cela ne constitue une restriction de l'invention. Les proportions (ou *« concentrations »* ou *« teneurs »)* en poids du principe actif seront par conséquent indiquées dans ce qui suit en référence audit Préventol®.

Ainsi, si l'on indique dans ce qui suit que la substance désinfectante 2 contient 5% en poids de principe actif phénolique, cela signifiera que ladite substance désinfectante 2 contient 5% de préparation de Préventol®, c'est à dire environ 3,6% de 3-méthyl-4-chlorophénolate de sodium pur.

Selon une variante de réalisation préférentielle, la substance désinfectante 2 comprend au moins un premier solvant de type alcool avec lequel est mélangé le principe actif phénolique, de manière à ce que ladite substance désinfectante 2 forme une solution volatile à température ambiante.

En effet, pour obtenir une bonne diffusion du principe actif phénolique en phase gazeuse dans l'atmosphère environnant le dispositif 1, il est nécessaire que la tension de vapeur de la substance désinfectante 2 soit particulièrement élevée.

A cette fin, on choisit tout d'abord de préférence un principe actif phénolique qui présente une tension de vapeur à 20°C élevée, et notamment supérieure à celle du Rhodoquat RP 80 ®. C'est en particulier le cas du Préventol®, dont la tension de vapeur à 20°C est de l'ordre de 8 kPa.

De plus, en dissolvant le Préventol® dans un solvant de type alcool, on obtient un liquide présentant une volatilité élevée, c'est à dire que l'on tend à modifier, et en l'occurrence à augmenter, la tension de vapeur du principe actif phénolique.

De façon particulièrement préférentielle, ledit premier solvant est de l'éthanol. Dans ce qui suit, on assimilera le premier solvant à de l'éthanol, sans que cela ne constitue une limitation de l'invention.

L'éthanol présente ici un double avantage : d'une part le Préventol® est soluble dans l'éthanol, et d'autre part l'éthanol est un composé chimique abondamment disponible et particulièrement bon marché.

Bien que la présente invention ne se limite pas à une utilisation particulière du dispositif 1, ce dernier est de préférence conçu et dimensionné pour la désinfection d'articles vestimentaires.

Ainsi, le dispositif 1 conforme à l'invention est de préférence destiné à être utilisé dans un milieu clos ou confiné pour désinfecter et désodoriser des objets amenés à être en contact avec la peau humaine, tels que des vêtements ou encore des équipements sportifs (chaussures, gants, casques et autres protections contre les chocs, etc.)

De façon particulièrement préférentielle, le dispositif 1 conforme à l'invention constitue un dispositif de désinfection pour chaussures, dont le volume de diffusion utile est généralement compris entre 500 $cm^3$ et 700 $cm^3$, et dont l'action est dirigée de préférence, mais pas exclusivement, contre la bactérie *Staphilococcus Epidermidis* (bactérie présente dans la chaussure) et la levure *Candida Albicans* (champignon présent dans la chaussure).

Selon une variante de réalisation préférentielle illustrée sur les figures 1 et 2, le dispositif 1 conforme à l'invention se présente sensiblement sous la forme d'une sphère, le moyen de diffusion 3 comprenant un organe de rétention 4 qui est conçu pour être imprégné de la substance désinfectante 2 en vue de diffuser progressivement le principe actif phénolique dans l'atmosphère.

De préférence, ledit organe de rétention 4 est formé par un élément spongieux et/ou poreux tel qu'un tampon de cellulose ou de coton apte à être imbibé de la substance désinfectante 2.

Selon la variante de réalisation préférentielle illustrée sur les figures 1 et 2, le dispositif de désinfection 1 comporte également un boîtier 5 qui est agencé pour contenir l'organe de rétention 4. Ledit boîtier 5 est de préférence sensiblement sphérique et pourvu d'une ou plusieurs ouvertures 6, ou *« évents »*, afin d'autoriser un échange entre l'organe de rétention 4 et l'air ambiant qui soit suffisant pour assurer l'efficacité de la désinfection.

Bien entendu, le dispositif conforme à l'invention n'est pas limité à une forme ni à des dimensions particulières de boîtier, d'ouvertures ni d'organe de rétention.

A titre d'exemple, on pourra réaliser l'organe de rétention 4 à l'aide d'une boule de cotillon de diamètre compris entre 18 mm et 22 mm, logée dans un boîtier de diamètre externe idoine et percé de trous circulaires mettant en communication l'intérieur du boîtier avec l'atmosphère.

Par ailleurs, il est envisageable que les ouvertures 6 soient réglables, de manière à ce que l'utilisateur puisse choisir la vitesse de diffusion du principe actif phénolique en réglant la surface d'échange et/ou le débit de circulation

d'air qui vient se charger en principe actif au contact de l'organe de rétention 4.

De préférence, la proportion en poids du principe actif phénolique (c'est à dire du Préventol®) dans la substance désinfectante 2 est sensiblement comprise entre 1 % et 20%, de façon plus préférentielle entre 3 % et 13 %, et de façon particulièrement préférentielle sensiblement égale à 5 (cette dernière valeur correspondant à environ 3,6 % de 3-méthyl-4-chlorophénolate de sodium pur).

Une proportion de 5 % de Préventol® s'est en effet révélée nettement suffisante pour satisfaire aux critères usuels d'efficacité bactéricide et fongicide.

En effet, des essais effectués conformément à la norme EN 1040 ont permis d'observer des réductions logarithmiques de souches de bactéries *Staphilococcus Epidermidis* de 5 log, que la substance désinfectante 2 soit utilisée pure, ou diluée au demi ou au quart.

De même, l'efficacité fongicide de la substance désinfectante 2 a été avérée sur une souche de *Candida Albicans* en suivant le protocole de la norme EN 1275 puisque des réductions de 4 log ont été constatées.

Il est par ailleurs remarquable que la teneur en Préventol® de la solution désinfectante 2 au sein du dispositif conforme à l'invention peut être ajustée en fonction de l'efficacité recherchée.

En particulier, il est envisageable que la solution désinfectante 2 contienne entre 5 % et 8 % en poids de Préventol®, voire 8 % à 13 % en poids pour améliorer l'efficacité dudit dispositif à encombrement constant.

A ce titre, les inventeurs ont toutefois constaté que l'augmentation de la concentration en Préventol® dans la substance désinfectante 2 tendait à fragiliser et à accélérer la dégradation des boîtiers 5 lorsque ceux-ci étaient réalisés en copolyester. En particulier, il a été observé des phénomènes de craquelures parfois dès la mise en contact de la substance 2 avec le boîtier.

En particulier, les inventeurs ont procédé à des tests de compatibilité en imprégnant un organe de rétention 4 sphérique en cellulose de 22 mm de diamètre ou deux organes de 18 mm et 22 mm de diamètre avec une substance désinfectante 2 contenant 5 % de Préventol® et en plaçant celui-ci au contact d'une coque en copolyester.

Lorsque l'on imprègne les organes de rétention 4 à 3 g et 2 g de la substance désinfectante 2, la coque se fissure rapidement et devient inutilisable.

*A contrario,* lorsque la quantité de substance désinfectante 2 contenant 5 % de Préventol® est réduite à 3 g, les coques en copolyester résistent en montrant une parfaite compatibilité après un mois à température ambiante de l'ordre de 20°C et à 40°C.

Une proportion de Préventol® de 5% en poids dans la solution désinfectante reste donc compatible avec l'utilisation de coques en copolyester, pourvu que la quantité totale de substance désinfectante utilisée (en masse) ne dépasse pas un seuil critique.

Par ailleurs, selon une variante de réalisation préférentielle, le boîtier 5 peut être formé par une coque en polypropylène, les inventeurs ayant découvert que ce matériau résiste particulièrement bien aux agressions du principe actif phénolique, et notamment ne présente pas de craquelures lorsque le Préventol® est employé à des concentrations élevées, même de l'ordre de 12 % ou 13 % en poids.

A titre indicatif, les tests de compatibilité ont démontré qu'en imprégnant deux organes de rétention 4 sphérique en cellulose de 22 mm de diamètre et de 18 mm avec 3 g puis de 2 g d'une substance désinfectante 2 conforme à l'invention contenant 12,8 % de Préventol®, et en maintenant le dispositif à une température de 40°C pendant 3 mois, aucune fêlure des coques en polypropylène n'est apparue, ce qui atteste que ces dernières sont compatibles avec une utilisation au sein d'un dispositif 1 conforme à l'invention.

Par ailleurs, la proportion en poids du premier solvant, et plus précisément de l'éthanol, dans la substance désinfectante 2, est de préférence sensiblement comprise entre sensiblement 25 % et 35 %, et de façon particulièrement préférentielle sensiblement égale à 30 %.

Le dosage de l'éthanol relève d'un compromis. En effet, l'éthanol favorise la diffusion aérienne du principe actif phénolique, ce qui incite à l'utiliser en proportion élevée dans la substance désinfectante 2, et en tout cas au moins à hauteur de 20% environ en poids pour obtenir une volatilité suffisante.

Toutefois, lorsque l'éthanol se trouve en excès, c'est à dire lorsque sa proportion en poids dans la substance désinfectante 2 dépasse un seuil critique, déterminé empiriquement comme voisin de 30 % à 35 % en poids, il modifie les propriétés de la substance désinfectante 2, et notamment sa densité, de telle sorte qu'il devient pratiquement impossible pour l'organe de rétention 4 de s'imprégner de ladite substance désinfectante 2 et de contenir celle-ci.

En d'autres termes, une solution désinfectante 2 trop riche en éthanol tendrait à s'échapper sous forme liquide du tampon de cellulose sans que celui-ci puisse la retenir.

Bien entendu, un tel comportement rhéologique est incompatible avec la fabrication comme avec l'usage normal d'un dispositif 1 conforme aux variantes illustrées sur les figures 1 et 2, ne serait-ce que parce que la substance désinfectante s'échapperait directement et très rapidement sous forme liquide par les ouvertures 6, au lieu de se diffuser progressivement dans l'atmosphère.

Selon une variante de réalisation particulièrement préférentielle, la substance désinfectante 2 comprend au moins un extrait parfumé, tel qu'une essence ou une huile essentielle.

A titre d'exemple, l'extrait parfumé pourra exhaler une odeur de menthe ou de lavande.

L'extrait parfumé pourra naturellement être employé pur ou fourni dilué sous la forme d'une préparation disponible auprès des parfumeurs.

De façon particulièrement avantageuse, l'effet olfactif obtenu grâce à l'adjonction d'un extrait parfumé dans la substance désinfectante 2 permet de masquer l'odeur du principe actif phénolique qui est généralement désagréable. La combinaison d'un effet désinfectant et d'un effet parfumant permet également de désodoriser plus efficacement la zone traitée en conférant une senteur plus « *fraîche* » à celle-ci.

De préférence, la proportion en poids de l'extrait parfumé dans la substance désinfectante 2 est sensiblement inférieure ou égale à 65 %, de façon particulièrement préférentielle comprise entre 20 % et 40 %, et de façon encore plus préférée sensiblement égale à 30 %. Cette dernière valeur permet en effet d'obtenir un effet olfactif jugé satisfaisant tout en limitant le surcoût de fabrication engendré par l'utilisation, même en quantité minime, d'une essence ou d'une huile essentielle.

Selon une variante de réalisation préférentielle, la substance désinfectante 2 comprend un second solvant dont la proportion en poids est sensiblement inférieure ou égale à 65%, de préférence comprise entre 25 % et 45 %, et de façon encore plus préférentielle sensiblement égale à 35 %.

Avantageusement, le second solvant et/ou l'extrait parfumé permettent d'ajuster les proportions en poids du principe actif phénolique et du premier solvant dans la substance désinfectante 2, ce qui permet notamment de ne pas dépasser la teneur critique en éthanol au-delà de laquelle la rétention de la substance désinfectante 2 par l'organe de rétention 4 devient problématique, et/ou la teneur en principe actif phénolique à partir duquel le boîtier 5 est fragilisé par les agressions de ladite substance désinfectante.

Bien entendu, le second solvant et l'extrait parfumé présentent de préférence une bonne compatibilité avec le boîtier 5, ainsi qu'une compatibilité chimique qui garantit leur miscibilité mutuelle et leur miscibilité avec le principe actif phénolique ainsi que le premier solvant.

Comme second solvant, il est envisageable d'employer du di-éthyl-phtalate (« *DEP* »).

Toutefois, des soupçons de toxicité pesant sur le DEP, on préférera que le second solvant soit majoritairement formé de di-propylène glycol (« *DPG* ») et, de façon particulièrement préférentielle, que le second solvant soit exclusivement formé de DPG.

De façon particulièrement avantageuse, le DPG est un solvant bon marché, dont l'innocuité est attestée, et qui est universellement répandu auprès des parfumeurs. Ainsi, il est notamment envisageable que ledit DPG serve de solvant à une huile essentielle, de sorte qu'il soit intégré à l'extrait parfumé.

Bien entendu, la présente invention n'est pas limitée à un second solvant particulier, lequel peut notamment intégrer d'autres composés, et par exemple être formé à base de DPG mélangé à du DEP, ce dernier étant présent selon une forme et une quantité compatibles avec les exigences légales en matière sanitaire et de sécurité des personnes.

Les inventeurs ont constaté qu'il était possible, dès lors que la base de la solution désinfectante 2 formée par le Préventol® et par l'éthanol était constituée, de diluer celle-ci en ajoutant une quantité suffisante soit du second solvant seul, soit d'un mélange du second solvant et de l'extrait parfumé.

Ainsi, si l'on note « *A* » la proportion en poids souhaitée du principe actif, « *E* » la proportion souhaitée en éthanol, « *S* » la proportion en second solvant et « *P* » la proportion en extrait parfumé, on obtient :

$$A + E + S + P = 100\%, \text{ c'est-à-dire : } [S + P] = 100\% - [A + E] \quad (i)$$

Les proportions A et E sont les plus contraignantes puisqu'elles conditionnent la fonctionnalité du dispositif 1. Toutefois, une fois fixées, elles permettent de définir une plage de valeurs au sein de laquelle les concentrations S et P sont susceptibles d'être choisie au gré du fabricant, pourvu que l'égalité (i) soit respectée.

Le tableau 1 fournit quelques exemples non limitatifs de formulations possibles 5 d'une substance désinfectante 2 conforme à l'invention (valeurs exprimées en % en poids).

*Tableau 1*

| Exemple | Préventol® (A) | Ethanol (E) | DPG (S) | Parfum (P) |
|---|---|---|---|---|
| 1 | 3 à 5 | 20 à 30 | 65 à 77 | 0 |
| 2 | 3 à 5 | 30 à 35 | 60 à 67 | 0 |
| 3 | 3 à 5 | 20 à 30 | 35 à 57 | 20 à 30 |
| 4 | 3 à 5 | 30 à 35 | 20 à 37 | 30 à 40 |

(suite)

| Exemple | Préventol® (A) | Ethanol (E) | DPG (S) | Parfum (P) |
|---|---|---|---|---|
| 5 | 5 à 7 | 20 à 30 | 63 à 75 | 0 |
| 6 | 5 à 7 | 30 à 35 | 58 à 65 | 0 |
| 7 | 5 à 7 | 20 à 30 | 33 à 55 | 20 à 30 |
| 8 | 5 à 7 | 30 à 35 | 18 à 35 | 30 à 40 |
| 9 | 10 à 13 | 20 à 30 | 57 à 70 | 0 |
| 10 | 10 à 13 | 30 à 35 | 52 à 60 | 0 |
| 11 | 10 à 13 | 20 à 30 | 27 à 50 | 20 à 30 |
| 12 | 10 à 13 | 30 à 35 | 12 à 30 | 30 à 40 |
| 13 | 10 à 13 | 25 à 35 | 0 à 25 | 40 à 52 |
| 14 | 12 à 15 | 25 à 35 | 0 à 23 | 30 à 60 |

Par ailleurs, la présente invention concerne également la substance désinfectante 2 proprement dite.

Ladite substance désinfectante 2 conforme à l'invention est conçue pour être diffusée dans l'atmosphère par évaporation naturelle et comprend, selon une caractéristique importante, un principe actif phénolique.

Plus précisément, comme cela a été détaillé plus haut, ledit principe actif phénolique est de préférence à base de 3-méthyl-4-chlorophénolate de sodium, et de préférence majoritairement, voire exclusivement, formé de ce dernier composé.

La substance désinfectante 2 conforme à l'invention pourra naturellement faire l'objet de toutes les variations détaillées précédemment.

A titre d'exemple de réalisation préférentielle, la substance désinfectante 2 conforme à l'invention pourra présenter sensiblement la composition suivante : 5 % de Préventol®, 30 % d'éthanol en tant que premier solvant, 30 % d'extrait parfumé et 35 % de di-propylène glycol en tant que second solvant.

Il est remarquable qu'il est parfaitement envisageable d'utiliser 65 % de DPG en tant que second solvant et de ne pas recourir à l'utilisation d'un extrait parfumé sans pour autant compromettre ni modifier significativement l'effet technique premier recherché, c'est-à-dire l'action bactéricide et fongicide de la substance désinfectante 2.

Il est également remarquable que la substance désinfectante 2 conforme à l'invention est de préférence sensiblement exempte d'eau.

Par ailleurs, la présente invention concerne également un procédé de fabrication d'un dispositif de désinfection par voie aérienne 1 dans lequel on associe une substance désinfectante 2 à un moyen de diffusion 3 conçu pour diffuser par évaporation naturelle ladite substance désinfectante dans l'atmosphère qui environne ledit dispositif 1, la substance désinfectante 2 employée comprenant, selon une caractéristique importante de l'invention, un principe actif phénolique.

De préférence, ledit principe actif phénolique est à base de 3-méthyl-4-chlorophénolate de sodium.

Le procédé de fabrication conforme à l'invention comprend de préférence une étape au cours de laquelle on imbibe un organe de rétention 4 formé par un corps spongieux de type tampon cellulosique, de manière à constituer un réservoir de substance désinfectante que l'on rapporte ensuite dans un boîtier 5.

Ledit procédé de fabrication comprend de préférence une étape au cours de laquelle on fabrique le boîtier 5 en réalisant une coque en polymère, de préférence en copolyester. Ladite coque peut avantageusement être formée par la réunion de deux demi-coques obtenues par exemple par moulage.

Enfin, l'invention concerne également un procédé d'élaboration d'une substance désinfectante 2 conçue pour être diffusée dans l'atmosphère par évaporation naturelle. Bien entendu, le procédé d'élaboration de la substance désinfectante 2, qui constitue un procédé de *« fabrication »*de ladite substance désinfectante, peut avantageusement être considéré comme une étape du procédé de fabrication du dispositif 1 sus-mentionné.

Selon une caractéristique importante de l'invention, le procédé d'élaboration de la substance désinfectante 2 comporte une étape (a) au cours de laquelle on réalise une préparation comprenant un principe actif phénolique.

Ledit principe phénolique est, au sens de l'invention, destiné à se retrouver dans la composition finale de ladite solution désinfectante 2.

De façon préférentielle, le principe actif phénolique utilisé est à base de 3-méthyl-4-chlorophénolate de sodium et est de préférence majoritairement, voire exclusivement, formé de ce composé. Le principe phénolique se présentera sous la forme de Préventol® solides, d'aspect d'écailles blanc à jaunâtre.

De préférence, le procédé d'élaboration comprend ensuite d'une étape (b) au cours de laquelle on incorpore,

notamment par dissolution, le Préventol® dans l'éthanol pour former un premier mélange.

On maintient alors le premier mélange sous agitation jusqu'à ce qu'il soit parfaitement homogénéisé.

De façon particulièrement préférentielle, on réalise au cours du procédé d'élaboration le premier mélange Préventol® - éthanol préalablement à l'adjonction de tout autre composant.

De préférence, on adjoint ensuite audit premier mélange, lors d'une étape (c), du DPG ou un mélange de DPG et d'essence parfumée pour obtenir un second mélange.

Ledit second mélange est maintenu sous agitation.

Ainsi, la substance désinfectante 2 et le dispositif de désinfection 1 conformes à l'invention permettent avantageusement de mettre en oeuvre une formulation bactéricide et fongicide active en phase aérienne qui peut se substituer aux produits de l'art antérieur, en présentant une efficacité au moins comparable ainsi qu'un meilleur respect de l'environnement comme de la santé des utilisateurs.

De façon particulièrement avantageuse, grâce à la sélection du principe actif et au dosage idoine des divers constituants de la substance désinfectante 2, le dispositif 1 conforme à l'invention permet d'obtenir des performances intéressantes en conciliant efficacité d'action, longévité accrue du fait d'une meilleure résistance du boîtier 5 formant son emballage, et absence de désagréments, notamment olfactifs, liés à sa mise en oeuvre.

## Revendications

1. - Dispositif de désinfection par voie aérienne (1) comprenant d'une part une substance désinfectante (2) et, d'autre part, un moyen de diffusion (3) conçu pour diffuser par évaporation naturelle ladite substance désinfectante (2) dans l'atmosphère qui environne ledit dispositif (1), **caractérisé en ce que** ladite substance désinfectante (2) comprend un principe actif phénolique.

2. - Dispositif selon la revendication 1 **caractérisé en ce que** le principe actif phénolique est à base de 3-méthyl-4-chlorophénolate de sodium.

3. - Dispositif selon la revendication 1 ou 2 **caractérisé en ce que** la substance désinfectante (2) comprend au moins un premier solvant de type alcool, avec lequel est mélangé le principe actif phénolique de manière à ce que ladite substance désinfectante (2) forme une solution volatile à température ambiante.

4. - Dispositif selon la revendication 3 **caractérisé en ce que** le premier solvant est de l'éthanol.

5. - Dispositif selon la revendication 3 ou 4 **caractérisé en ce que** le moyen de diffusion (3) comprend un organe de rétention (4), tel qu'un tampon de cellulose, conçu pour être imprégné de la substance désinfectante (2) en vue de diffuser progressivement le principe actif phénolique dans l'atmosphère.

6. - Dispositif selon la revendication 5 **caractérisé en ce qu'**il comporte un boîtier (5) agencé pour contenir l'organe de rétention (4), de préférence sensiblement sphérique, ledit boîtier étant pourvu d'une ou plusieurs ouvertures (6) afin d'autoriser un échange entre l'organe de rétention (4) et l'air ambiant.

7. - Dispositif selon la revendication 6 **caractérisé en ce que** le boîtier (5) est formé par une coque en copolyester.

8. - Dispositif selon la revendication 6 **caractérisé en ce que** le boîtier (5) est formé par une coque en polypropylène.

9. - Dispositif selon l'une des revendications précédentes **caractérisé en ce que** la proportion en poids du principe actif phénolique dans la substance désinfectante (2) est sensiblement comprise entre 1 % et 20 %, de préférence entre 3 % et 13 %, et de façon particulièrement préférentielle sensiblement égale à 5 %.

10. - Dispositif selon la revendication 4 **caractérisé en ce que** la proportion en poids de l'éthanol dans la substance désinfectante est sensiblement comprise entre sensiblement 25 % et 35 %, et de préférence sensiblement égale à 30 %.

11. - Dispositif selon l'une des revendications précédentes **caractérisé en ce que** la substance désinfectante comprend au moins un extrait parfumé, tel qu'une essence ou une huile essentielle.

12. -Dispositif selon la revendication 11 **caractérisé en ce que** la proportion en poids de l'extrait parfumé dans la substance désinfectante est sensiblement inférieure ou égale à 65 %, de préférence comprise entre 20 % et 40 %,

et de façon encore plus préférentielle sensiblement égale à 30 %.

**13.** - Dispositif selon l'une des revendications précédentes **caractérisé en ce que** la substance désinfectante comprend un second solvant dont la proportion en poids est sensiblement inférieure ou égale à 65 %, de préférence comprise entre 25 % et 45 %, et de façon encore plus préférentielle sensiblement égale à 35 %.

**14.** Dispositif selon la revendication 13 **caractérisé en ce que** le second solvant est majoritairement formé de dipropylène glycol.

**15.** - Dispositif selon l'une des revendications précédentes **caractérisé en ce qu'**il est conçu pour la désinfection d'articles vestimentaires, et plus particulièrement de chaussures.

**16.** - Substance désinfectante conçue pour être diffusée dans l'atmosphère par évaporation naturelle, **caractérisée en ce qu'**elle comprend un principe actif phénolique.

**17.** - Substance désinfectante selon la revendication 16 **caractérisée en ce que** le principe actif phénolique est à base de 3-méthyl-4-chlorophénolate de sodium.

**18.** - Substance désinfectante selon la revendication 16 ou 17 **caractérisée en ce qu'**elle comprend un premier solvant pour augmenter la tension de vapeur du principe actif phénolique.

**19.** - Substance désinfectante selon la revendication 18 **caractérisée en ce qu'**elle comprend au moins un premier solvant de type alcool avec lequel est mélangé le principe actif phénolique, de manière à ce que ladite substance désinfectante forme une solution volatile à température ambiante.

**20.** - Substance désinfectante selon la revendication 18 ou 19 **caractérisée en ce que** le premier solvant comprend de l'éthanol, la proportion en poids de l'éthanol dans la substance désinfectante ne dépassant pas un seuil critique, de préférence égal à 35 % en poids, au-delà duquel il devient pratiquement impossible pour un organe de rétention de s'imprégner de ladite substance désinfectante et de contenir celle-ci.

**21.** - Substance désinfectante selon la revendication 16 ou 17 **caractérisée en ce que** le principe actif phénolique présente une tension de vapeur à 20°C de l'ordre de 8 kPa.

**22.** - Substance désinfectante selon la revendication 21 **caractérisée en ce que** le principe actif phénolique est disponible sous le nom commercial *« PREVENTOL CMK-NA® ».*

**23.** - Substance désinfectante selon l'une des revendications 16, 17, 21 ou 22 **caractérisée en ce que** la proportion en poids du principe actif phénolique dans la substance désinfectante est sensiblement comprise entre 3 % et 13 %, et de façon particulièrement préférentielle sensiblement égale à 5 %.

**24.** - Procédé de fabrication d'un dispositif de désinfection par voie aérienne, dans lequel on associe une substance désinfectante à un moyen de diffusion, conçu pour diffuser par évaporation naturelle ladite substance désinfectante dans l'atmosphère qui environne ledit dispositif, **caractérisé en ce que** la substance désinfectante employée comprend un principe actif phénolique.

**25.** - Procédé de fabrication selon la revendication 24 **caractérisé en ce que** le principe actif phénolique est à base de 3-méthyl-4-chlorophénolate de sodium.

**26.** - Procédé d'élaboration d'une substance désinfectante conçue pour être diffusée dans l'atmosphère par évaporation naturelle, **caractérisé en ce qu'**il comporte une étape au cours de laquelle on réalise une préparation comprenant un principe actif phénolique.

**27.** - Procédé d'élaboration selon la revendication 26 **caractérisé en ce que** le principe actif phénolique utilisé est à base de 3-méthyl-4-chlorophénolate de sodium.

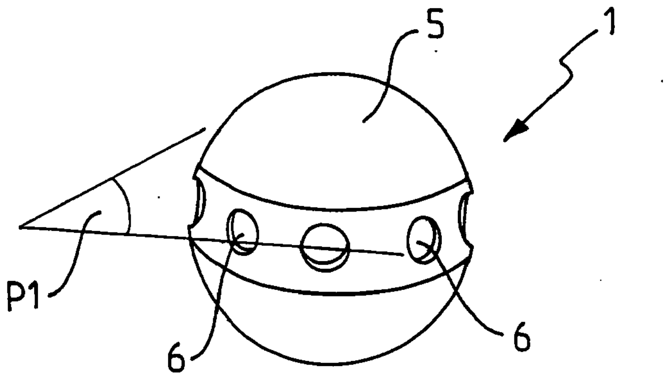


FIG.1

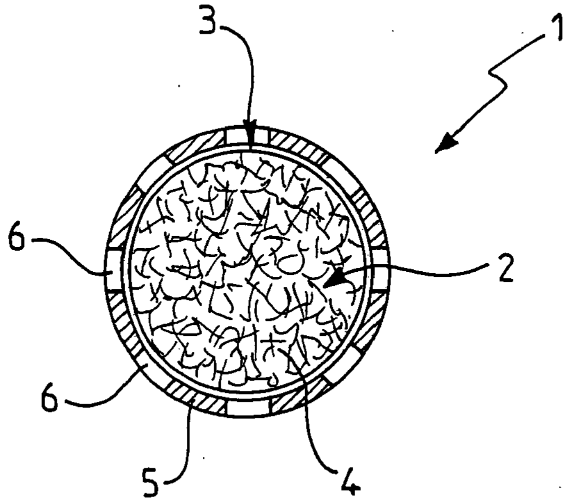


FIG.2

Office européen des brevets

# RAPPORT DE RECHERCHE EUROPEENNE

Numéro de la demande
EP 07 35 6117

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 2 793 692 A1 (VASSEUR PIERRE [FR]) 24 novembre 2000 (2000-11-24) * revendications; figure 1; exemple 1 * | 1-15, 24-27 | INV. A61L2/20 A61L9/04 C07C39/28 |
| X | DE 103 18 009 A1 (MENNO CHEMIE VERTRIEBSGES MBH [DE]) 18 novembre 2004 (2004-11-18) | 16-23 | |
| Y | * revendication 1 * | 1-15, 24-27 | |
| X | US 5 863 562 A (TSAO FU-PAO [US] ET AL) 26 janvier 1999 (1999-01-26) * colonne 4, ligne 8 - ligne 22 * | 16-18 | |
| A | WO 2004/100666 A (ARCH UK BIOCIDES LTD [GB]; HODGE DAVID JOHN [GB]; PEARS DAVID ALAN [GB) 25 novembre 2004 (2004-11-25) * page 28, ligne 11 * | 1-27 | |

DOMAINES TECHNIQUES RECHERCHES (IPC)

A61L
C07C

Le présent rapport a été établi pour toutes les revendications

| Lieu de la recherche | Date d'achèvement de la recherche | Examinateur |
|---|---|---|
| Munich | 21 janvier 2008 | Haderlein, Andreas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

3

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 35 6117

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-01-2008

| Document brevet cité au rapport de recherche | | Date de publication | Membre(s) de la famille de brevet(s) | | | Date de publication |
|---|---|---|---|---|---|---|
| FR 2793692 | A1 | 24-11-2000 | AUCUN | | | |
| DE 10318009 | A1 | 18-11-2004 | AUCUN | | | |
| US 5863562 | A | 26-01-1999 | AU | 710353 | B2 | 16-09-1999 |
| | | | AU | 5249896 | A | 12-12-1996 |
| | | | CA | 2177538 | A1 | 01-12-1996 |
| | | | EP | 0745391 | A1 | 04-12-1996 |
| | | | JP | 8322911 | A | 10-12-1996 |
| | | | NO | 962182 | A | 02-12-1996 |
| | | | NZ | 286677 | A | 28-07-1998 |
| | | | US | 5611464 | A | 18-03-1997 |
| | | | ZA | 9604369 | A | 02-12-1996 |
| WO 2004100666 | A | 25-11-2004 | EP | 1624754 | A1 | 15-02-2006 |
| | | | JP | 2007505988 | T | 15-03-2007 |

EPO FORM P0460

Pour tout renseignement concernant cette annexe : voir Journal Officiel de l'Office européen des brevets, No.12/82